# EUROPÄISCHE PATENTSCHRIFT

(11) **EP 0 596 304 B1**
(45) Veröffentlichungstag und Bekanntmachung des Hinweises auf die Patenterteilung: **01.04.1998**
(21) Anmeldenummer: 93116746.4
(22) Anmeldetag: 16.10.1993
(51) Int. Cl.: A61K 7/48, A61K 7/00, A61K 7/06, A61K 47/34

(54) **Kosmetische oder pharmazeutische Zubereitungen**
Cosmetic and pharmaceutical compositions
Compositions cosmétiques et pharmaceutiques

(30) Priorität: 31.10.1992 DE 4236861
(43) Veröffentlichungstag der Anmeldung: 11.05.1994
(73) Patentinhaber: Th. Goldschmidt AG, 45127 Essen (DE)
(72) Erfinder: Grüning, Burghard, Dr., D-45134 Essen (DE); Weitemeyer, Christian, Dr., D-45134 Essen (DE)

(56) Entgegenhaltungen:
- EP-A- 0 310 903
- EP-A- 0 435 483
- EP-A- 0 495 596
- EP-A- 0 549 223
- FR-A- 2 686 510
- US-A- 3 641 239

## Beschreibung

Die Erfindung betrifft kosmetische oder pharmazeutische Zubereitungen mit einem Gehalt an Estergruppen enthaltenden natürlichen oder synthetischen Ölen oder Mineralölen mit verbessertem Spreitungsverhalten.

Es ist bekannt, daß Polysiloxane die Spreitung von Ölen auf Oberflächen, wie z.B. der menschlichen Haut, aber auch auf Oberflächen von Kunststoffen verbessern. Zu diesem Stand der Technik wird auf die EP-OS 0 211 550 verwiesen. Diese betrifft eine Zusammensetzung, welche folgendes enthält:
(A) 5 bis 95 Gew.-% eines alkoxylierten Polyethers der Formel 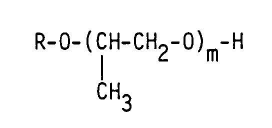 worin R für eine gesättigte oder ungesättigte C₆₋₂₂-Kohlenwasserstoff-Gruppe steht, die durch eine Hydroxyl-Gruppe substituiert sein kann, und m für 2 bis 50 steht, und
(B) 95 bis 5 Gew.-% Tetra(dimethylcyclosiloxan) oder Penta(dimethylcyclosiloxan) oder ein Gemisch davon.

Derartige Siliconöl enthaltende Polyether können in Form von Cremes oder Lotionen verwendet werden. Sie können aber auch als Träger für physiologisch wirksame Substanzen, einschließlich pharmazeutischer Substanzen, verwendet werden. Die Haupteigenschaft einer solchen Zubereitung besteht in der Verbesserung ihrer Spreitungsfähigkeit auf der Haut. Die Spreitungsfähigkeit hängt unter anderem und insbesondere von der Oberflächenspannung der das Silicon enthaltenden Zubereitung ab, wobei in der EP-OS 0 211 550 auf den Aufsatz von R. Keymer: "Zur Spreitung flüssiger Lipoide auf der Haut", Pharm. Ind. 32 (7), 577 - 581 (1970) verwiesen wird.

Gemäß EP-A-0 435 483 werden spezielle Emulsionen zur Auftragung auf Haut oder Haare vorgeschlagen. Dabei handelt es sich um Wasser-in-Silikonöl-Emulsionen, die als Emulgator ein mit Polyoxyalkylengruppen modifiziertes Polydimethylsiloxan enthalten.

Die Verwendung von alkylmodifizierten Siloxanen zum Verdicken von Ölen ist aus EP-A-0 310 903 bekannt. Auch ihr Einsatz in kosmetischen Formulierungen ist aus dem Stand der Technik bekannt. So werden beispielsweise gemäß EP-A-0 495 596 filmbildende Formulierungen für die menschliche Haut beschrieben, die deren Wasserverlust vermindern soll und anstelle von reinen Polydimethylsiloxanen als filmbildende Komponente ein Alkylmethyl-Polysiloxan-Copolymer enthält.

In EP-A-0 549 223 werden Deostift-Formulierungen beschrieben, die nichtflüchtige, ggf. alkylmethylmodifizierte Siloxane neben üblichen Wirk- und Hilfsstoffen als Hauptbestandteil enthalten.

US-A-3 641 239 beschreibt eine kosmetische Formulierung auf Wachs-Basis, insbesondere Lippenstift-Formulierungen, die u. a. ein organisches Wachs und ein Silikonwachs enthalten, wobei letzteres u. a. ein alkylmodifiziertes Polysiloxan sein kann.

Der Einsatz von öligen Formulierungen mit verbessertem Spreitungsverhalten in kosmetischen oder pharmazeutischen Zubereitungen ist keiner dieser Dokumente zu entnehmen.

Eine weitere Klasse von Spreitungsmitteln stellen Siliconwachse dar, welche am Silicongerüst gebunden einen oder mehrere langkettige Alkylreste aufweisen. Mit steigendem Gehalt an langkettigen Alkylgruppen sowie mit steigender Kettenlänge der Alkylgruppen steigt der Schmelzpunkt der Siliconwachse an. Hierzu wird auf die Veröffentlichung "Silicone Surfactants" von D. Schaefer in Tenside 1990, Seiten 154 bis 158, verwiesen. Diese Siliconwachse erniedrigen die Oberflächenspannung organischer Systeme, wie z.B. die Oberflächenspannung von Mineralölen, und verbessern die Spreitungsfähigkeit kosmetischer Öle und Wachse. Die Mischungen von Ölen und Siliconwachsen weisen, verglichen mit den reinen Ölen, in der Regel jedoch nur eine um ein geringes verbesserte Spreitungsfähigkeit auf.

Ein Nachteil der als Mittel zur Verbesserung der Spreitungsfähigkeit verwendeten Polysiloxane besteht in ihrer meist schlechten Verträglichkeit mit Ölen, wobei die Verträglichkeit (Löslichkeit) der Polysiloxane mit den Ölen mit steigender Anzahl an Dimethylsiloxyeinheiten abfällt.

Die Erfindung befaßt sich mit dem technischen Problem, Organopolysiloxane aufzufinden, welche eine möglichst gute Verträglichkeit (Löslichkeit) in organischen Ölen aufweisen und die Fähigkeit besitzen, das Spreitungsvermögen der Öle auf Oberflächen, insbesondere der menschlichen Haut, zu verbessern. Die Verbindungen sollen in möglichst geringer Menge deutlich wirksam sein und in der Lage sein, auch Öle unterschiedlicher Struktur bezüglich ihres Spreitungsverhaltens zu verbessern.

Ein Gegenstand der vorliegenden Erfindung sind deshalb ölige kosmetische oder pharmazeutische Zubereitungen mit verbessertem Spreitungsverhalten auf der Basis von Estergruppen enthaltenden natürlichen oder synthetischen Ölen oder Mineralölen, gekennzeichnet durch einen Gehalt an Organopolysiloxanen der allgemeinen Formel 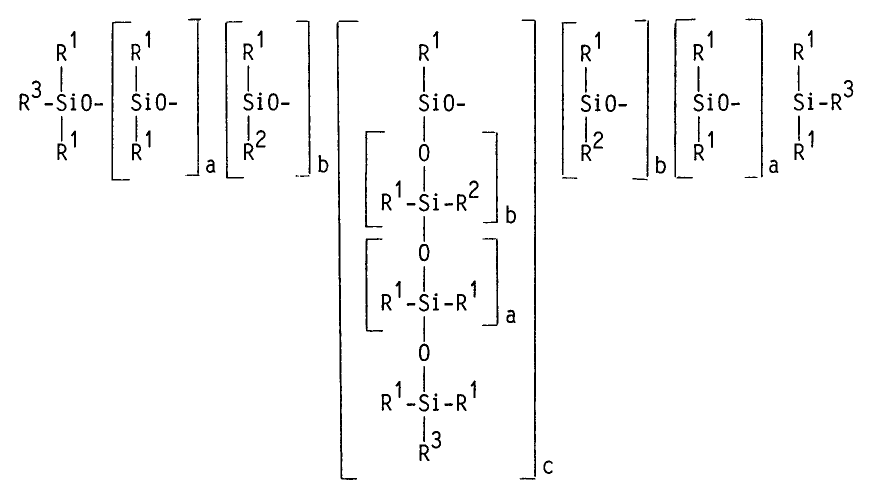 wobei die Reste
- R¹: in dem Polymeren gleich oder verschieden sind und Alkylreste mit 1 bis 4 Kohlenstoffatomen bedeuten, jedoch mindestens 90 % der Reste R¹ Methylreste sind,
- R²: in dem Polymeren gleich oder verschieden sind und
(1) geradkettige oder verzweigte Alkylreste mit 8 bis 30 Kohlenstoffatomen, wobei die Alkylreste durch ein Sauerstoffatom unterbrochen sein können oder
(2) geradkettige oder verzweigte Alkoxyreste mit 8 bis 30 Kohlenstoffatomen, wobei der Alkylrest der Alkoxyreste durch ein Sauerstoffatom unterbrochen sein kann, sind,
- R³: die Bedeutung der Reste R¹ oder R² hat,
a jeweils einen Wert von 5 bis 100,
b jeweils einen Wert von 0 bis 80 und
c einen Wert von 0 bis 5 hat,
mit der Maßgabe, daß
(i) im durchschnittlichen Molekül wenigstens 2 Reste R² die Bedeutung (1) und/oder (2) haben,
(ii) a > b ist und die Summe der Einheiten mit dem Index a > 10 ist und
(iii) das Organopolysiloxan mit dem Öl in einer Konzentration von 0,01 bis 20 Gew.-% homogen mischbar ist,
in Mengen von 0,003 bis 20 Gew.-%, bezogen auf Gesamtgewicht von Öl und Organopolysiloxan.

In der Formel I können die Reste R¹ in dem Polymeren gleich oder verschieden sein. Sie sind Alkylreste mit 1 bis 4 Kohlenstoffatomen und können geradkettig oder verzweigt sein. Beispiele solcher Reste sind Methyl-, Ethyl-, Propyl-, Isopropyl-, Butyl- oder Isobutylreste.

Mindestens 90 % der Reste R¹ müssen Methylreste sein, wobei die Angabe 90 % als Zahlenangabe zu verstehen ist. Vorzugsweise sind alle Reste R¹ Methylreste.

Die Reste R² sind Reste, mit welchen die Organopolysiloxane modifiziert werden und welche die Verträglichkeit mit den Ölen bewirken.

Die Reste R² können dabei im Polymeren gleich oder verschieden sein und die folgenden Bedeutungen haben:
(1) geradkettige oder verzweigte Alkylreste mit 8 bis 30 Kohlenstoffatomen, wobei die Alkylreste durch ein Sauerstoffatom unterbrochen sein können oder
(2) geradkettige oder verzweigte Alkoxyreste mit 8 bis 30 Kohlenstoffatomen, wobei der Alkylrest der Alkoxyreste durch ein Sauerstoffatom unterbrochen sein kann.

Dabei müssen jedoch die Bedingungen erfüllt sein, daß im durchschnittlichen Molekül wenigstens 2 Reste R² die Bedeutung (1) und/oder (2) haben.

Als geradkettige oder verzweigte Alkylreste (2) mit 8 bis 30 Kohlenstoffatomen kommen z.B. 2-Ethylhexyl-, Octyl-, Isonoyl-, Decyl-, Undecyl-, 2-Butloctyl-, Dodecyl-, Behenyl- und 2-Decyltetradecylreste sowie die entsprechenden Alkoxyreste in Frage. Bevorzugte Reste sind der Dodecyl-, Isotridecyl-, 2-Hexadecyl-, Hexadecyl-, Octadecyl- und 2-Decyltetradecylrest sowie die entsprechenden Alkoxyreste.

Unter dem Begriff "Alkylreste, welche durch ein Sauerstoffatom unterbrochen sein können" sollen im Rahmen dieser Erfindung Reste vom Typ -R¹-O-R'' (R¹ zweiwertiger aliphatischer Rest, R'' einwertiger aliphatischer Rest) verstanden werden. Ein solcher Rest ist zum Beispiel der Rest -(CH₂)₃-OC₁₂H₁₅. Weitere Beispiele sind der Isotridecyloxypropyl-, Hexadecyloxypropyl-, Octadecyloxyhexyl- und Dodecyloxyundecylrest.

Analoges gilt für den Begriff "Alkoxyrest, dessen Alkylrest durch ein Sauerstoffatom unterbrochen sein kann". Hierdurch sollen Reste der allgemeinen Formel -O-R'-O-R'' erfaßt werden (R' und R'' wie vorstehend definiert). Ein Beispiel ist der Rest -O-(CH₂)₂-O-C₁₂H₁₅.

Die Reste R² werden in das Organopolysiloxanmolekül meist in der Weise eingeführt, daß man die entsprechenden Wasserstoffsiloxane mit Olefinen oder ungesättigten Ethern hydrosilylierend umsetzt, wobei die Doppelbindung der Olefine endständig ist und die Kohlenstoffanzahl der Olefine der des gewünschten Restes R² entspricht.

Die Alkyl- oder die durch ein Sauerstoffatom unterbrochenen Alkylreste können aber auch über ein Sauerstoffatom mit jeweils einem Si-Atom des Polysiloxangerüstes verbunden sein. Diese Alkoxyreste können in das Molekül des Polysiloxans durch Umsetzung mit einer SiH- oder SiCl-Gruppe eingeführt werden.

Der Index a bezeichnet die Anzahl der Dialkylsiloxyeinheiten und hat einen Wert von 5 bis 100. Es handelt sich, da mindestens 90 % der Reste R¹ Methylreste sein müssen, demnach überwiegend um Dimethylsiloxyeinheiten. Die Summe der Einheiten mit dem Index a muß größer als 10 sein. Dies bedeutet, daß, wenn a = 5 ist, c nicht den Wert 0 annehmen kann.

Der Index b gibt die Anzahl der difunktionellen Siloxyeinheiten an, welche den Rest R² aufweisen. Der Index b hat einen Wert von 0 bis 80. Da aber die Bedingung gilt, daß im durchschnittlichen Molekül mindestens 2 Reste R² die Bedeutung (1) oder (2) haben müssen, müssen, wenn b = 0 ist, die beiden endständigen Reste R³ die Bedeutung von R²-Resten annehmen.

Der Index c gibt die Anzahl der verzweigenden trifunktionellen Siloxyeinheiten an. c hat einen Wert von 0 bis 5. Ist c = 0, hat b vorzugsweise einen Wert von 0 bis 20.

R³ kann die Bedeutung des Restes R¹ oder R² annehmen, wobei, wie bereits ausgeführt, R³ die Bedeutung des Restes R² haben muß, wenn der Index b = 0 ist.

Die in der erfindungsgemäßen Zubereitung enthaltenen Organopolysiloxane müssen eine weitere Bedingung erfüllen: Sie müssen mit dem jeweils in der erfindungsgemäßen Zubereitung enthaltenen Öl in einer Konzentration von 0,01 bis 20 Gew.-% bezogen auf das Gesamtgewicht von Öl und Organopolysiloxan, mischbar sein. Es ist dem Fachmann ohne weiteres zuzumuten, durch Variation der Kettenlänge der Reste R² mit der Bedeutung (1) und (2) sowie durch unterschiedliche Werte der Indices a und b ein erfindungsgemäßes Organopolysiloxan auszuwählen, welches diese Bedingung der Mischbarkeit erfüllt.

Dabei hat sich gezeigt, daß Öle auf der Basis von Estergruppen enthaltenden natürlichen oder synthetischen Ölen in ihrem Spreitungsvermögen durch Organopolysiloxane verbessert werden, welche entsprechend dem Molekulargewicht der Öle ausgewählt werden.

Ein bevorzugter Gegenstand vorliegender Erfindung besteht deshalb darin, daß die erfindungsgemäße Zubereitung als Öle Estergruppen aufweisende natürliche oder synthetische Öle enthält und die Organopolysiloxane entsprechend dem Molekulargewicht der Öle ausgewählt sind, wobei bei einem Molekulargewicht der Öle von
(i) > 500 die Summe der Einheiten mit dem Index a = 12 bis 35,
   die Summe der Einheiten mit dem Index b = 3 bis 10 und der Index c = 0 ist,
(ii) > 400 und < 500 die Summe der Einheiten mit dem Index a = 25 bis 60,
   die Summe der Einheiten mit dem Index b = 5 bis 15 und der Index c = 0 ist,
(iii) < 400 die Summe der Einheiten mit dem Index a = 45 bis 100,
   die Summe der Einheiten mit dem Index b = 3 bis 20 und der Index c = 0 ist.

Diese Auswahlregeln zeigen, daß mit fallendem Molekulargewicht des Öles die Anzahl der Einheiten mit dem Index a und b ansteigt.

Eine ähnliche Auswahl ist für Öle auf der Basis von Mineralölen möglich, wobei anstelle des Molekulargewichtes als Bemessungsgröße die Viskosität ausgewählt wird.

Ein weiterer Gegenstand der Erfindung besteht deshalb darin, daß die erfindungsgemäße Zubereitung als Öle Mineralöle enthält und die Organopolysiloxane entsprechend der Viskosität der Öle ausgewählt sind, wobei bei einer Viskosität der Öle bei 25°C von
(i) > 50 mPas die Summe der Einheiten mit dem Index a = 12 bis 60,
   die Summe der Einheiten mit dem Index b = 3 bis 15 und der Index c = 0 ist,
(ii) < 50 mPas die Summe der Einheiten mit dem Index a = 26 bis 100,
   die Summe der Einheiten mit dem Index b = 3 bis 20 und der Index c = 0 ist.

Im folgenden werden konkrete Organopolysiloxane der allgemeinen Formel I gezeigt, wobei zusätzlich angegeben wird, für welche Öle die jeweiligen Verbindungen zur Verbesserung der Spreitung der Öle besonders geeignet sind. 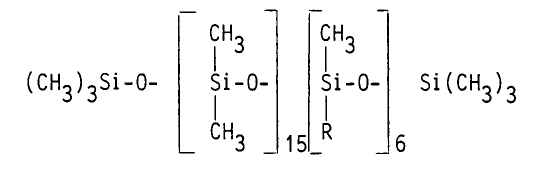
R = CH₃-(CH₂)₁₅-
für Esteröle mit Molgewichten > 500 und Mineralöle mit Viskositäten > 50 mPas (25°C). 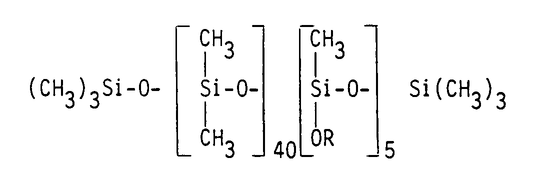
R = 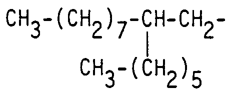
für Esteröle mit Molgewichten 400 bis 500 und Mineralöle mit Viskositäten > 50 mPas (25°C). 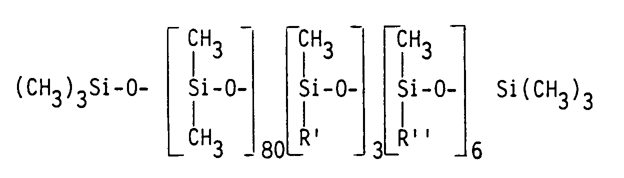
R' = CH₃-(CH₂)₁₁-
R'' = CH₃-(CH₂)₁₇-
für Esteröle mit Molgewichten < 400 und Mineralöle mit Viskositäten < 50 mPas (25°C).

Als Öle auf der Basis von Estergruppen enthaltenden natürlichen oder synthetischen Ölen sind die aus dem Stand der Technik bekannten flüssigen oder sich bei der Anwendung verflüssigenden Öle geeignet, wie z.B. Esteröle, tierische Öle sowie pflanzliche Öle und Fette.

Beispiele von Esterölen sind Decyloleat, Diethylhexyladipat, Pentaerythrittetraethylhexanoat. Als tierische oder pflanzliche Öle und Fette sind die Ester des Glycerins mit meist natürlich vorkommenden Fettsäuren geeignet. Ein Beispiel hierfür ist Avocadoöl. Es kommen jedoch auch die vollständigen oder partiellen Ester höherfunktioneller Alkohole, wie z.B. des Sorbits, in Frage. Ein weiteres Beispiel für verwendbare Öle ist Jojobaöl.

Beispiele von geeigneten Mineralölen sind verzweigte Kohlenwasserstofföle, Paraffinöle unterschiedlicher Viskosität, niedrigschmelzende Wachse und im weiteren Sinne Squalen und Squalan.

Die in den erfindungsgemäßen Zubereitungen enthaltenen Öle, welche die ausgewählten Organopolysiloxane der Formel I enthalten, zeigen ein hervorragendes Spreitungsverhalten bereits bei sehr niedrigen Gehalten an Organopolysiloxanen. Diese Organopolysiloxane werden in Mengen von 0,003 bis 20 Gew.-% bezogen auf Gesamtgewicht von Öl und Organopolysiloxan, den Ölen zugesetzt, wobei ein Gehalt von 0,01 bis 5 Gew.-%, insbesondere ein Gehalt von 0,1 bis 2 Gew.-% besonders bevorzugt ist.

Die kosmetischen oder pharmazeutischen Zubereitungen gemäß der Erfindung können in Form der die ausgewählten Organopolysiloxane enthaltenden Öle oder in Form von Lösungen oder Emulsionen verwendet werden. Die Emulsionen können dabei die Form einer Creme oder einer Lotion haben. Sie können in Form einer O/W- oder W/O-Emulsion vorliegen. Es ist auch möglich, diese Öle als Trägersubstanzen für Wirkstoffe zu verwenden. Beispiele von Wirkstoffen sind Farbpigmente, Duftstoffe, kosmetisch und/oder pharmazeutisch wirksame Substanzen, wie z.B. Vitamin E oder Nicotinsäureester.

Öle, welche Organopolysiloxane der Formel I enthalten, eignen sich besonders zur Herstellung hochwirksamer Sonnenschutzpräparate.

Der Gegenstand der Erfindung wird in den folgenden Beispielen näher erläutert. Dabei wird in Beispiel 1 die Beeinflussung des Spreitungsvermögens von Ölen durch die erfindungsgemäß zu verwendenden Organopolysiloxane und in den Beispielen 2 bis 6 werden die Zusammensetzung, Herstellung und Eigenschaften von 10 kosmetischen Emulsionen gezeigt.

In den Beispielen 2 bis 6 sind die Rezepturen paarweise geordnet. Jedes Paar ist mit I und II gekennzeichnet. Der Unterschied besteht jeweils in der Verwendung einer Ölphase mit verbesserter Spreitfähigkeit in der Rezeptur II, die ansonsten mit der jeweiligen Rezeptur I identisch ist. Die Rezepturen II stellen erfindungsgemäße Rezepturen dar, die Rezepturen I werden zum Vergleich angeführt.

### Beispiel 1

Die mittleren Formeln der verwendeten Alkylsiloxane sind nachfolgend aufgeführt, sie werden abgekürzt mit A bis G bezeichnet. Die Lösungen mit den Alkylsiloxanen A und B sind nicht erfindungsgemäß, sie werden lediglich zu Vergleichszwecken herangezogen. Die Lösungen mit den Alkylsiloxanen C bis G sind erfindungsgemäß.

Alkylsiloxan Formel A, nicht erfindungsgemäße Vergleichssubstanz: 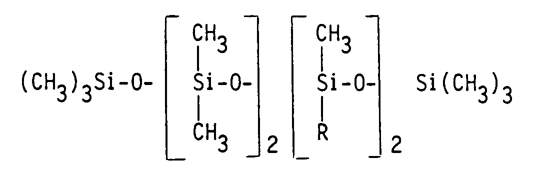
R = CH₃-(CH₂)₁₅-

Alkylsiloxan Formel B, nicht erfindungsgemäße Vergleichssubstanz:

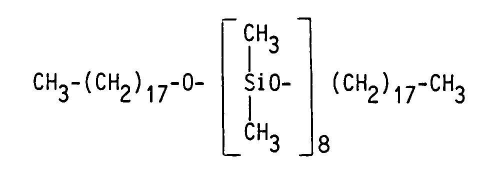

Alkylsiloxan Formel C, erfindungsgemäß: 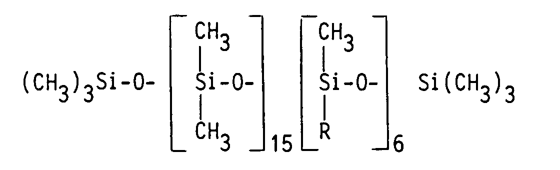
R = CH₃-(CH₂)₁₅-

Alkylsiloxan Formel D, erfindungsgemäß: 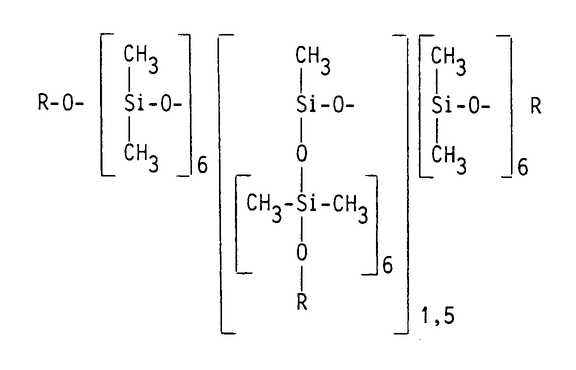
R =

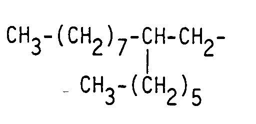

Alkylsiloxan Formel E, erfindungsgemäß: 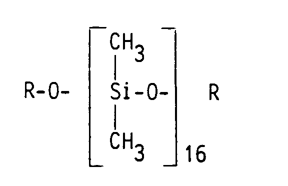
R =

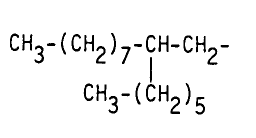

Alkylsiloxan Formel F, erfindungsgemäß: 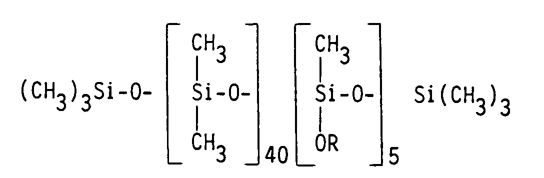
R =

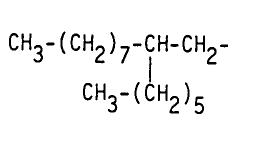

Alkylsiloxan Formel G, erfindungsgemäß: 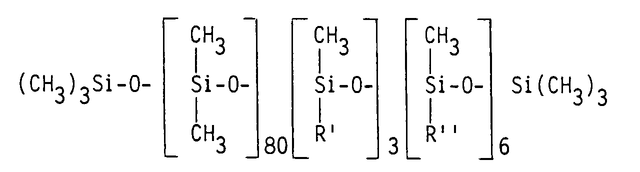
R' = CH₃-(CH₂)₁₁-
R'' = CH₃-(CH₂)₁₇-

Die Löslichkeit der Alkylsiloxane wird vorab geprüft, indem versucht wird, Lösungen mit 0,1 und 5 Gew.-% des Siloxans in dem jeweiligen Öl herzustellen. Die Ergebnisse sind in den Tabellen 1 und 2 zusammengestellt.

Löslichkeitsverhalten verschiedener Alkylsiloxane bei 0,1 Gew.-% in Ölen

**Tabelle 1**

| Öl | Alkylsiloxan | | | | | | |
|---|---|---|---|---|---|---|---|
| | A | B | C | D | E | F | G |
| 2-Ethylhexyl-2-ethylhexanat | l | l | l | l | l | l | l |
| Isopropylmyristat | l | l | l | l | l | l | l |
| Di-2-ethylhexyladipat | l | l | l | l | l | l | l |
| Cetyl-2-ethylhexanat | l | l | l | l | l | l | l |
| Decyloleat | l | l | l | l | l | l | l |
| 2-Hexyldecylpalmitat | l | l | l | l | l | l | l |
| Glycerintricapryl/caprinat | l | l | l | l | l | l | u |
| Jojobaöl | l | l | l | l | l | l | u |
| Avocadoöl | l | l | l | l | l | l | u |
| Paraffinöl [ Viskosität (25°C) 38 mPas ] | l | l | l | l | l | l | l |
| Paraffinöl [ Viskosität (25°C) 230 mPas ] | l | l | l | l | l | l | u |
| l = löslich, u = unlöslich | | | | | | | |

Löslichkeitsverhalten verschiedener Alkylsiloxane bei 5 Gew.-% in Ölen

**Tabelle 2**

| Öl | Alkylsiloxan | | | | | | |
|---|---|---|---|---|---|---|---|
| | A | B | C | D | E | F | G |
| 2-Ethylhexyl-2-ethylhexanat | l | l | l | l | l | l | l |
| Isopropylmyristat | l | l | l | l | l | l | l |
| Di-2-ethylhexyladipat | l | l | l | l | l | l | l |
| Cetyl-2-ethylhexanat | l | l | l | l | l | l | l |
| Decyloleat | l | l | l | l | l | l | u |
| 2-Hexyldecylpalmitat | l | l | l | l | l | l | u |
| Glycerintricapryl/caprinat | l | l | l | l | l | l | u |
| Jojobaöl | l | l | l | u | u | u | u |
| Avocadoöl | l | l | l | u | u | u | u |
| Paraffinöl [ Viskosität (25°C) 38 mPas ] | l | l | l | l | l | l | l |
| Paraffinöl [ Viskosität (25°C) 230 mPas ] | l | l | l | l | l | l | u |
| l = löslich, u = unlöslich | | | | | | | |

Die Spreitung der Alkylsiloxane wird auf zwei verschiedene Materialien, nämlich auf Polypropylen und auf Gelatine, überprüft.

Zur Herstellung der Gelatineschichten werden auf Glasplatten von 5 x 5 cm mit Hilfe einer Pipette jeweils 1 ml einer 1 %igen wäßrigen Gelatinelösung gleichmäßig verteilt. Unter Kühlung auf 0 bis 5°C wird die Gelatineschicht verfestigt, und anschließend werden die beschichteten Platten bei 25°C und 65 % rel. Luftfeuchte für 3 Tage gelagert.

Die verwendete Polypropylenfolie ist im Handel erhältlich (Forco-OPPB/AT-OPAL-Folie, Fa. 4P Folie Forchheim GmbH, Deutschland).

Zur Bestimmung der Spreitung werden 10 µl des Öles bzw. der Lösung auf eine Gelatine-beschichtete Glasplatte oder auf die Polypropylenfolie bei 25°C und 65 % rel. Luftfeuchte gegeben. Nach 10 Min. wird die benetzte Fläche ausgemessen. Die mit Esterölen auf Gelatine erhaltenen Resultate sind in Tabelle 3 aufgeführt. Die Alkylsiloxane werden mit 0,1 Gew.-% eingesetzt. Die mit Mineralölen auf Polypropylen und Gelatine erhaltenen Ergebnisse sind in den Tabellen 4 und 5 zusammengestellt. Die Alkylsiloxane werden hier mit 5 Gew.-% eingesetzt. Die Spreitflächen sind in mm² angegeben.

Esteröle mit 0,1 Gew.-% Alkylsiloxanzusatz, Spreitung auf Gelatine

**Tabelle 3**

| Öl | Alkylsiloxan | | | | | | | |
|---|---|---|---|---|---|---|---|---|
| | kein | A | B | C | D | E | F | G |
| 2-Ethylhexyl-2-ethylhexanat | 253 | 260 | 228 | 263 | 258 | 247 | 298 | 756 |
| Isopropylmyristat | 195 | 192 | 169 | 231 | 267 | 227 | 293 | 702 |
| Di-2-ethylhexyladipat | 186 | 175 | 175 | 192 | 201 | 194 | 220 | 236 |
| Cetyl-2-ethylhexanat | 163 | 146 | 122 | 161 | 182 | 163 | 172 | 274 |
| Decyloleat | 72 | 68 | 92 | 154 | 163 | 150 | 172 | 165 |
| 2-Hexyldecylpalmitat | 45 | 46 | 45 | 74 | 58 | 60 | 111 | 112 |
| Glycerintricapryl/caprinat | 96 | 88 | 101 | 158 | 153 | 163 | 165 | u |
| Jojobaöl | 50 | 57 | 65 | 95 | 95 | 60 | 102 | u |
| Avocadoöl | 48 | 45 | 52 | 87 | 50 | 50 | 85 | u |
| u = unlöslich | | | | | | | | |

Mineralöle mit 5 Gew.-% Alkylsiloxanzusatz, Spreitung auf Polypropylen

**Tabelle 4**

| Öl | Alkylsiloxan | | | | | |
|---|---|---|---|---|---|---|
| | kein | C | D | E | F | G |
| Paraffinöl (38 mPas) | 131 | 226 | 207 | 218 | 227 | 227 |
| Paraffinöl (230 mPas) | 64 | 104 | 108 | 95 | 101 | u |
| u = unlöslich | | | | | | |

Mineralöle mit 5 Gew.-% Alkylsiloxanzusatz, Spreitung auf Gelatine

**Tabelle 5**

| Öl | Alkylsiloxan | | | | | |
|---|---|---|---|---|---|---|
| | kein | C | D | E | F | G |
| Paraffinöl (38 mPas) | 52 | 99 | 113 | 140 | 88 | 135 |
| Paraffinöl (230 mPas) | 31 | 82 | 57 | 86 | 77 | u |
| u = unlöslich | | | | | | |

Den Tabellen ist zu entnehmen, daß mit den nicht erfindungsgemäßen Siloxanen A und B zwar in einigen Fällen eine geringe, in der Regel jedoch keine Spreitungsverbesserung zu erzielen ist. Einige Öle werden durch den Zusatz gar in ihrer Spreitfähigkeit vermindert.

Die Mischungen mit den Alkylsiloxanen C bis G (erfindungsgemäß) sind im Gegensatz dazu alle in ihrer Spreitfähigkeit gegenüber den reinen Ölen deutlich verbessert. Im allgemeinen werden die besten Resultate mit den höhermolekularen Alkylsiloxanen, insbesondere mit dem Alkylsiloxan G erzielt, jedoch ist andererseits zu berücksichtigen, daß in gleicher Richtung die Löslichkeit des Siloxans abnimmt. Um eine optimale Verbesserung der Spreitwirkung zu erzielen, ist daher eine wohl abgewogene Wahl des Siloxans in Abstimmung auf das jeweilige Öl notwendig. Als Auswahlkriterien hierzu können das Molekulargewicht bzw. die Viskosität des Öles herangezogen werden.

### Beispiel 2

### Wasser-in-Öl-Emulsion, geeignet als Nachtcreme

Es werden zwei Nachtcremes beschrieben, die sich in der Zusammensetzung der Ölphase unterscheiden. Der Unterschied besteht in dem zusätzlichen Einsatz eines Alkylsiloxans in der Rezeptur II. Das Alkylsiloxan entspricht der Formel C (vgl. Beispiel 1).

| Rezeptur (Angaben in %) | | I | II |
|---|---|---|---|
| Ölphase: | Triglycerintrioleat | 4,0 | 4,0 |
| | Bienenwachs | 1,5 | 1,5 |
| | Castorwax | 1,5 | 1,5 |
| | Glycerintricaprylat/caprinat | 12,5 | 12,0 |
| | Avocadoöl | 12,5 | 12,0 |
| | Alkylsiloxan C | - | 1,0 |
| Wasserphase: | Magnesiumsulfat Heptahydrat | 0,6 | 0,6 |
| | Glycerin | 2,0 | 2,0 |
| | 2-Brom-2-nitropropan-1,3-diol ¹⁾ | 0,1 | 0,1 |
| | Wasser | 65,3 | 65,3 |

| | | | |
|---|---|---|---|
| 1) Konservierungsmittel | | | |

Zur Herstellung der Creme wird die Wasserphase, die eine Temperatur von 25°C aufweist, in die auf 85°C erwärmte Ölphase unter intensivem Rühren eingebracht. Anschließend wird mit einer Kolloidmühle homogenisiert.

Die nach Rezeptur II hergestellte Creme weist im Vergleich zu der nach Rezeptur I hergestellten verbesserte Applikationseigenschaften auf. Sie läßt sich leicht verteilen, die Haut wirkt glatter und weniger fettig.

### Beispiel 3

### Wasser-in-Öl-Emulsion, geeignet als Sonnenschutzcreme

Es werden zwei Sonnenschutzcremes beschrieben, die sich hinsichtlich der Zusammensetzung der Ölphase unterscheiden. Der Unterschied besteht in dem zusätzlichen Einsatz eines Alkylsiloxans in der Rezeptur II. Das Alkylsiloxan entspricht der Formel G (vgl. Beispiel 1).

| Rezeptur (Angaben in %) | | I | II |
|---|---|---|---|
| Ölphase: | Abil EM 90 ¹⁾ | 2,5 | 2,5 |
| | Ceresin ²⁾ | 1,0 | 1,0 |
| | Castorwax | 0,5 | 0,5 |
| | Isohexadecan | 7,0 | 7,0 |
| | Octylmethoxycinnamat | 3,0 | 3,0 |
| | Octylstearat | 13,0 | 12,75 |
| | Titandioxiddispersion ³⁾ | 10,0 | 10,0 |
| | Alkylsiloxan G | - | 0,25 |
| Wasserphase: | Natriumchlorid | 0,5 | 0,5 |
| | 2-Brom-2-nitropropan-1,3-diol ⁴⁾ | 0,1 | 0,1 |
| | Wasser | 62,4 | 62,4 |

| | | | |
|---|---|---|---|
| 1) siliciumorganischer Emulgator für W/O-Emulsionen, Handelsprodukt der Th. Goldschmidt AG | | | |
| 2) mikrokristallines Kohlenwasserstoffwachs | | | |
| 3) bestehend aus 30 % ultrafeinem TiO₂ in Octylpalmitat | | | |
| 4) Konservierungsmittel | | | |

Die Cremes werden nach der in Beispiel 2 angegebenen Vorschrift hergestellt.

Die Sonnenschutzwirkung der Rezepturen I und II wurde in vivo an Menschen gemäß DIN 67 501 untersucht. Dabei wurden für die Rezeptur I ein SPF (sun protection factor) von 8,9 und für die Rezeptur II ein SPF von 13,7 erhalten. Die Ergebnisse belegen die überlegene Wirksamkeit der Rezeptur II, die aufgrund des Zusatzes des Alkylsiloxans G eine Ölphase mit verbesserter Spreitwirkung enthält.

### Beispiel 4

### Öl-in-Wasser-Emulsion, geeignet als Sonnenschutzcreme

Es werden zwei Sonnenschutzcremes beschrieben, die sich hinsichtlich der Zusammensetzung der Ölphase unterscheiden. Der Unterschied besteht in dem zusätzlichen Einsatz eines Alkylsiloxans in der Rezeptur II. Das Alkylsiloxan entspricht der Formel G (vgl. Beispiel 1).

| Rezeptur (Angaben in %) | | I | II |
|---|---|---|---|
| Ölphase: | TEGO Care 450 ¹⁾ | 2,0 | 2,0 |
| | Octylpalmitat | 6,5 | 6,5 |
| | Paraffinöl ²⁾ | 6,5 | 6,0 |
| | Octylmethoxycinnamat | 4,0 | 4,0 |
| | 2-Hydroxy-4-methoxybenzophenon | 1,0 | 1,0 |
| | Alkylsiloxan G | - | 0,5 |
| Wasserphase: | Glycerin | 3,0 | 3,0 |
| | Carbopol 941 ³⁾ | 20,0 | 20,0 |
| | 2-Brom-2-nitropropan-1,3-diol ⁴⁾ | 0,1 | 0,1 |
| | Wasser | 56,9 | 56,9 |

| | | | |
|---|---|---|---|
| 1) Triglycerin-/Methylglucosedistearat, Emulgator für O/W-Emulsionen, Handelsprodukt der Th. Goldschmidt AG | | | |
| 2) Viskosität 30 mPas | | | |
| 3) Verdickungsmittel, Handelsprodukt der Firma Goodrich | | | |
| 4) Konservierungsmittel | | | |

Zur Herstellung der Cremes werden die Öl- und Wasserphase auf 70 bis 75°C erwärmt, bei dieser Temperatur zusammengegeben und unter Verwendung eines Rotor-Stator-Rührers homogenisiert bis die Tröpfchen eine Größe von 3 bis 5 µm aufweisen. Anschließend wird unter langsamem Rühren auf 30°C abgekühlt.

Die Sonnenschutzwirkung der Rezepturen I und II wurde in vivo an Menschen gemäß DIN 67 501 untersucht. Dabei wurden für die Rezeptur I ein SPF von 5,2 und für die Rezeptur II ein SPF von 7,0 erhalten. Die Ergebnisse belegen die bessere Wirksamkeit der Rezeptur II, die aufgrund des Zusatzes des Alkylsiloxans G eine Ölphase mit verbesserter Spreitwirkung enthält.

### Beispiel 5

### Öl-in-Wasser-Emulsion, geeignet als Tagescreme

Es werden zwei Rezepturen beschrieben, die sich hinsichtlich der Zusammensetzung der Ölphase unterscheiden. Der Unterschied besteht in dem zusätzlichen Einsatz eines Alkylsiloxans in der Rezeptur II. Das Alkylsiloxan entspricht der Formel F (vgl. Beispiel 1).

| Rezeptur (Angaben in %) | | I | II |
|---|---|---|---|
| Ölphase: | TEGIN ¹⁾ | 6,0 | 6,0 |
| | Stearinsäure | 2,0 | 2,0 |
| | Octylpalmitat | 7,0 | 7,0 |
| | Hexadecylpalmitat | 10,0 | 8,5 |
| | Alkylsiloxan F | - | 1,5 |
| Wasserphase: | Glycerin | 1,9 | 1,9 |
| | 2-Brom-2-nitropropan-1,3-diol ²⁾ | 0,1 | 0,1 |
| | Wasser | 75,0 | 75,0 |

| | | | |
|---|---|---|---|
| 1) selbstemulgierendes Glycerinmono/distearat, Handelsprodukt der Th. Goldschmidt AG | | | |
| 2) Konservierungsmittel | | | |

Zur Herstellung der Cremes werden die Öl- und Wasserphase auf 65 bis 70°C erwärmt und bei dieser Temperatur zusammengegeben. Anschließend wird in der gleichen Weise wie in Beispiel 4 beschrieben verfahren.

Die nach Rezeptur II hergestellte Creme weist im Vergleich zu der nach Rezeptur I hergestellten verbesserte Applikationseigenschaften auf. Sie läßt sich leicht verteilen, die Haut wirkt geschmeidiger und weniger stumpf.

### Beispiel 6

### Öl-in-Wasser-Emulsion, geeignet als Bodylotion

Es werden zwei Rezepturen beschrieben, die sich hinsichtlich der Zusammensetzung der Ölphase unterscheiden. Der Unterschied besteht in dem zusätzlichen Einsatz eines Alkylsiloxans in der Rezeptur II. Das Alkylsiloxan entspricht der Formel G (vgl. Beispiel 1)

| Rezeptur (Angaben in %) | | I | II |
|---|---|---|---|
| Ölphase: | PEG-15-Stearylalkohol | 1,5 | 1,5 |
| | Glycerinmono/distearat | 1,0 | 1,0 |
| | Stearylalkohol | 2,0 | 2,0 |
| | Octylpalmitat | 5,5 | 5,0 |
| | Paraffinöl ¹⁾ | 5,0 | 5,0 |
| | Alkylsiloxan G | - | 0,5 |
| Wasserphase: | Glycerin | 2,9 | 2,9 |
| | 2-Brom-2-nitropropan-1,3-diol ²⁾ | 0,1 | 0,1 |
| | Wasser | 82,0 | 82,0 |

| | | | |
|---|---|---|---|
| 1) selbstemulgierendes Glycerinmono/distearat, Handelsprodukt der Th. Goldschmidt AG | | | |
| 2) Konservierungsmittel | | | |

Zur Herstellung der Cremes werden die Öl- und Wasserphase auf 75 bis 80°C erwärmt und bei dieser Temperatur zusammengegeben. Anschließend wird in der gleichen Weise wie in Beispiel 4 beschrieben verfahren.

Die nach Rezeptur II hergestellte Creme weist im Vergleich zu der nach Rezeptur I hergestellten verbesserte Applikationseigenschaften auf. Sie läßt sich leicht verteilen, während des Verteilens wird Weißeln verhindert, die Haut wirkt geschmeidiger und weniger wachsartig stumpf.

## Patentansprüche

1. Ölige kosmetische oder pharmazeutische Zubereitungen mit verbessertem Spreitungsverhalten auf der Basis von Estergruppen enthaltenden natürlichen oder synthetischen Ölen oder Mineralölen, gekennzeichnet durch einen Gehalt an Organopolysiloxanen der allgemeinen Formel 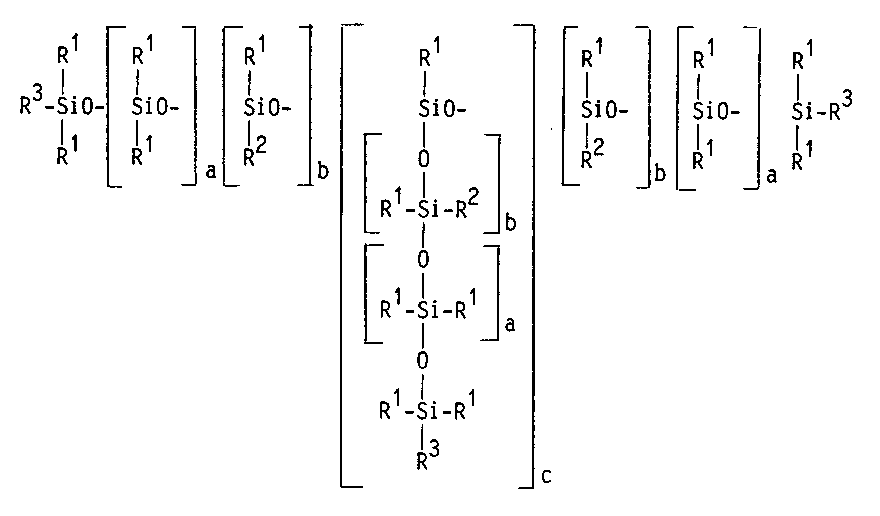 wobei die Reste
R¹ in dem Polymeren gleich oder verschieden sind und Alkylreste mit 1 bis 4 Kohlenstoffatomen bedeuten, jedoch mindestens 90 % der Reste R¹ Methylreste sind,
R² in dem Polymeren gleich oder verschieden sind und
(1) geradkettige oder verzweigte Alkylreste mit 8 bis 30 Kohlenstoffatomen, wobei die Alkylreste durch ein Sauerstoffatom unterbrochen sein können oder
(2) geradkettige oder verzweigte Alkoxyreste mit 8 bis 30 Kohlenstoffatomen, wobei der Alkylrest der Alkoxyreste durch ein Sauerstoffatom unterbrochen sein kann, sind,
R³ die Bedeutung der Reste R¹ oder R² hat,
a jeweils einen Wert von 5 bis 100,
b jeweils einen Wert von 0 bis 80 und
c einen Wert von 0 bis 5 hat,
mit der Maßgabe, daß
(i) im durchschnittlichen Molekül wenigstens 2 Reste R² die Bedeutung (1) und/oder (2) haben,
(ii) a > b ist und die Summe der Einheiten mit dem Index a > 10 ist und
(iii) das Organopolysiloxan mit dem Öl in einer Konzentration von 0,01 bis 20 Gew.-% homogen mischbar ist,
in Mengen von 0,003 bis 20 Gew.-%, bezogen auf Gesamtgewicht von Öl und Organopolysiloxan.

2. Zubereitung nach Anspruch 1, dadurch gekennzeichnet, daß sie ein Organopolysiloxan enthält, dessen Index b einen Wert von 0 bis 20 und c einen Wert von 0 hat.

3. Zubereitung nach Anspruch 1 oder 2, dadurch gekennzeichnet, daß sie als Öle Estergruppen aufweisende natürliche oder synthetische Öle enthält und die Organopolysiloxane entsprechend dem Molekulargewicht der Öle ausgewählt sind, wobei bei einem Molekulargewicht der Öle von
(i) > 500 die Summe der Einheiten mit dem Index a = 12 bis 35,
die Summe der Einheiten mit dem Index b = 3 bis 10 und der Index c = 0 ist,
(ii) > 400 und < 500 die Summe der Einheiten mit dem Index a = 25 bis 60,
die Summe der Einheiten mit dem Index b = 5 bis 15 und der Index c = 0 ist,
(iii) < 400 die Summe der Einheiten mit dem Index a = 45 bis 100,
die Summe der Einheiten mit dem Index b = 3 bis 20 und der Index c = 0 ist.

4. Zubereitung nach Anspruch 1 oder 2, dadurch gekennzeichnet, daß sie als Öle Mineralöle enthält und die Organopolysiloxane entsprechend der Viskosität der Öle ausgewählt sind, wobei bei einer Viskosität der Öle bei 25°C von
(i) > 50 mPas die Summe der Einheiten mit dem Index a = 12 bis 60,
die Summe der Einheiten mit dem Index b = 3 bis 15 und der Index c = 0 ist,
(ii) < 50 mPas die Summe der Einheiten mit dem Index a = 26 bis 100,
die Summe der Einheiten mit dem Index b = 3 bis 20 und der Index c = 0 ist.

5. Zubereitung gemäß den Ansprüchen 1 bis 4, dadurch gekennzeichnet, daß sie als Emulsion vorliegt.

## Claims

1. Oily cosmetic or pharmaceutical preparations having improved spreading behaviour based on natural or synthetic oils or mineral oils containing ester groups, characterized by a content of organopolysiloxanes of the general formula 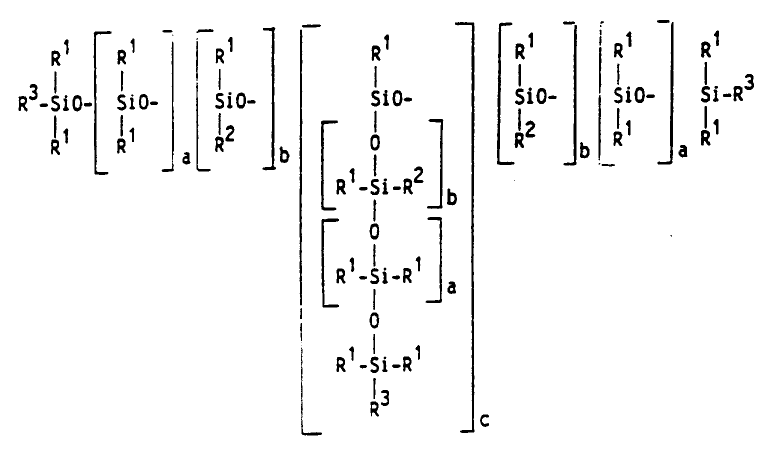 where the radicals
R¹ in the polymer are identical or different and are alkyl radicals having from 1 to 4 carbon atoms, but at least 90% of the R¹ radicals are methyl radicals,
R² in the polymer are identical or different and
(1) are linear or branched alkyl radicals having from 8 to 30 carbon atoms, where the alkyl radicals may be interrupted by an oxygen atom, or
(2) are linear or branched alkoxy radicals having from 8 to 30 carbon atoms, where the alkyl radical of the alkoxy radicals may be interrupted by an oxygen atom,
R³ is as defined for the R¹ or R² radical,
a is in each case a value from 5 to 100,
b is in each case a value from 0 to 80 and
c is a value from 0 to 5,
with the proviso that
(i) in the average molecule at least 2 R² radicals are as defined under (1) and/or (2),
(ii) a > b and the sum of the units having the index a is > 10 and
(iii) the organopolysiloxane is homogeneously miscible with the oil in a concentration of from 0.01 to 20% by weight,
in quantities of from 0.003 to 20% by weight, based on the total weight of oil and organopolysiloxane.

2. Preparation according to Claim 1, characterized in that it contains an organopolysiloxane whose index b has a value of from 0 to 20 and c has a value of 0.

3. Preparation according to Claim 1 or 2, characterized in that the oils present are natural or synthetic oils having ester groups, and the organopolysiloxanes are chosen according to the molecular weight of the oils, and for a molecular weight of the oils of
(i) > 500 the sum of the units having the index a = 12 to 35,
the sum of the units having the index b = 3 to 10 and the index c = 0,
(ii) > 400 and < 500 the sum of the units having the index a = 25 to 60,
the sum of the units having the index b = 5 to 15 and the index c = 0,
(iii) < 400 the sum of the units having the index a = 45 to 100,
the sum of the units having the index b = 3 to 20 and the index c = 0.

4. Preparation according to Claim 1 or 2, characterized in that the oils present are mineral oils, and the organopolysiloxanes are chosen according to the viscosity of the oils, and for a viscosity of the oils at 25°C of
(i) > 50 mPas the sum of the units having the index a = 12 to 60,
the sum of the units having the index b = 3 to 15 and the index c = 0,
(ii) < 50 mPas the sum of the units having the index a = 26 to 100,
the sum of the units having the index b = 3 to 20 and the index c = 0.

5. Preparation according to Claims 1 to 4, characterized in that it is in the form of an emulsion.

## Revendications

1. Préparations huileuses cosmétiques ou pharmaceutiques ayant un comportement à l'étalement amélioré, à base d'huiles minérales ou d'huiles naturelles ou synthétiques contenant des groupes ester, caractérisées en ce qu'elles possèdent une teneur en organopolysiloxanes de formule générale : 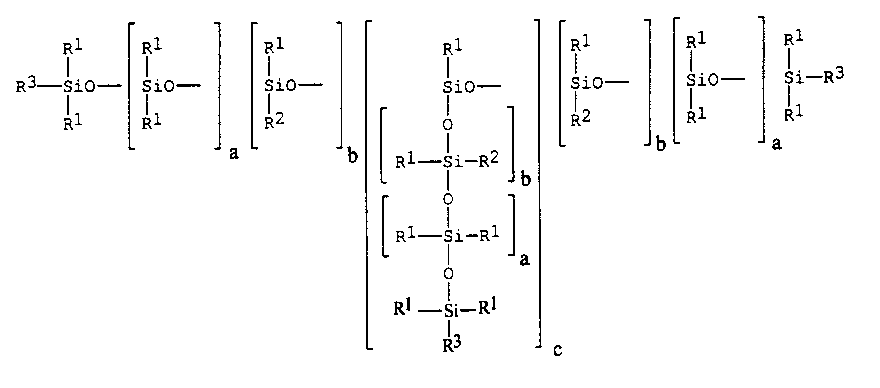 dans laquelle :
les radicaux R¹ dans le polymère sont identiques ou différents, et signifient des radicaux alcoyle avec 1 à 4 atomes de carbone, au moins 90% des radicaux R¹ étant toutefois des radicaux méthyle;
les radicaux R² dans le polymère sont identiques ou différents, et
(1) sont des radicaux alcoyle linéaires ou ramifiés avec 8 à 30 atomes de carbone, les radicaux alcoyle pouvant être interrompus par un atome d'oxygène, ou
(2) sont des radicaux alcoxy linéaires ou ramifiés avec 8 à 30 atomes de carbone, le radical alcoyle des radicaux alcoxy pouvant être interrompu par un atome d'oxygène;
R³ a la signification des radicaux R¹ ou R²;
a vaut dans chaque cas de 5 à 100;
b vaut dans chaque cas de 0 à 80, et
c vaut dans chaque cas de 0 à 5,
sous réserve que
(i) dans une molécule moyenne, au moins 2 radicaux R² aient la signification (1) et/ou (2);
(ii) a > b et la somme des unités ayant l'indice a > 10, et
(iii) l'organopolysiloxane est miscible de manière homogène à l'huile à une concentration de 0,01 à 20% en poids,
dans des quantités de 0,003 à 20% en poids, par rapport au poids total de l'huile et de l'organopolysiloxane.

2. Préparation suivant la revendication 1, caractérisée en ce qu'elle contient un organopolysiloxane dont l'indice b vaut de 0 à 20 et dont l'indice c vaut 0.

3. Préparation suivant la revendication 1 ou 2, caractérisée en ce qu'elle contient comme huiles des huiles naturelles ou synthétiques présentant des groupes ester et en ce que les organopolysiloxanes sont choisis en fonction du poids moléculaire des huiles, où, avec un poids moléculaire des huiles de :
(i) >500, la somme des unités d'indice a = 12 à 35;
la somme des unités d'indice b = 3 à 10, et l'indice c = 0,
(ii) >400 et <500, la somme des unités d'indice a = 25 à 60,
la somme des unités d'indice b = 5 à 15, et l'indice c = 0,
(iii) <400 la somme des unités d'indice a = 45 à 100;
la somme des unités d'indice b = 3 à 20, et l'indice c = 0.

4. Préparation suivant la revendication 1 ou 2, caractérisée en ce qu'elle contient comme huiles des huiles minérales et en ce que les organopolysiloxanes sont choisis en fonction de la viscosité des huiles, de sorte que, avec une viscosité des huiles à 25°C de :
(i) >50 mPas la somme des unités d'indice a = 12 à 60,
la somme des unités d'indice b = 3 à 15, et l'indice c = 0,
(ii) <50 mPas la somme des unités d'indice a = 26 à 100,
la somme des unités de l'indice b = 3 à 20, et l'indice c = 0.

5. Préparation suivant les revendications 1 à 4, caractérisée en ce qu'elle se présente sous forme d'émulsion.
